(19) Europäisches Patentamt
European Patent Office
Office européen des brevets

(11) **EP 3 726 220 A1**

(12) **EUROPEAN PATENT APPLICATION**

(43) Date of publication:
**21.10.2020 Bulletin 2020/43**

(51) Int Cl.:
*G01N 33/574* [(2006.01)]     *G01N 33/68* [(2006.01)]

(21) Application number: **19000185.9**

(22) Date of filing: **15.04.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Institut Clinident SAS 13592 Aix en Provence Cedex 3 (FR)**

(72) Inventor: **Chaubron, Franck 3770 Venelles (FR)**

(74) Representative: **Barbot, Willy Simodoro-ip 82, rue Sylvabelle 13006 Marseille (FR)**

(54) **USE OF VOLATILE ORGANIC COMPOUNDS SIGNATURE TO IMPROVE THE DIAGNOSIS OF ORAL CANCER**

(57)     The present invention relates to method of diagnosing oral cancer or assessing risk of developing oral cancer in a subject, comprising detecting in a saliva sample obtained from the subject the presence of a VOC signature comprising four ratios indicative of oral cancer or risk of developing oral cancer and comparing said ratios to reference ratios, wherein a difference in ratios for said VOCs indicates an oral cancer or a risk of developing oral cancer.

**FIGURE 1**

**Description**

**FIELD OF THE INVENTION:**

The present invention relates to the identification of a unique combination of volatile organic compounds (VOCs) useful for the diagnosis or oral cancer.

**BACKGROUND OF THE INVENTION:**

The annual incidence of head and neck cancers worldwide is more than 600,000 cases with around 300,000 deaths each year. About 90% of all head and neck cancers are squamous cell carcinomas (HNSCC). Most HNSCCs arise in the epithelial lining inside of the mouth. HNSCC is classified by its location: it can occur in the mouth (oral cavity), the middle part of the throat near the mouth (oropharynx), the space behind the nose (nasal cavity and paranasal sinuses), the upper part of the throat near the nasal cavity (nasopharynx), the voice box (larynx), or the lower part of the throat near the larynx (hypopharynx). These cancers are strongly associated with risk factors like tobacco and alcohol consumption, which can act both independently and synergistically, and more recently, to human papilloma virus infection.

Due to an asymptomatic first phase of development, the diagnosis of head and neck cancers often occurs relatively late. Currently, lesions involving the larynx and pharynx are detected thorough oral examination. A detailed history and physical examination including complete head and neck examination with biopsy is necessary to establish the diagnosis of head and neck cancers, depending on the tumor location and appearance. Molecular signature based on the identification of genetic and epigenetic biomarkers in biopsy is helpful in diagnosing of head and neck cancers. However, these techniques require a tissue sample and their implementation takes time. A more easily accessible type of sample would be a considerable additional help for the practitioner. Saliva, a watery and frothy substance produced in the mouth, meets the demand for an inexpensive, non invasive and accessible diagnostic fluid for identifying biological markers relevant for the disease. HU *et al.* identified five proteins in saliva of patient suffering from oral squamous cell carcinoma that collectively provide a signature indicative of oral cancer (Clin. Cancer Res., vol.14, p:6246-6252, 2008). In addition to the presence of proteins, saliva also contains volatile organic compounds (VOCs). In the European patent EP 2 321 430 B1, the analysis of the volatile fraction of saliva issued from patient with oral cancer allows the identification of 14 volatile organic compounds overexpressed in oral cancer population. Statistical models relying on the linear combination of five variable ratios allows to discriminate samples of oral cancer patients and healthy volunteers. Nevertheless, some of the VOCs used in these statistical models are suspected to arise artefactually from analysis conditions and/or from external contamination.

As a consequence, there is a need to identify a VOCs signature, i.e. a unique combination of VOCs, to distinguish head and neck cancer patients from a group of healthy control subject.

**SUMMARY OF THE INVENTION:**

The inventors have previously demonstrated that it is possible to extract biochemical organic compounds from the volatile fraction of stabilized saliva samples. A link between fourteen organic compounds and oral cancer in human was thus established for the first time.

The inventors have now surprisingly found that specific combinations of VOCs ratio can significantly improve the diagnostic of oral cancer. Combined testing of VOCs ratio is able to increase the sensitivity to almost 92% and serial combined examination offered a specificity of over 95%.

Consequently, the present invention relates to an *in vitro* method of diagnosing oral cancer or assessing the risk of developing oral cancer in a subject, comprising:

a) determining the concentration of at least the seven following volatile organic compounds (VOCs) in a saliva sample obtained from the subject:

- 2-methyl-2-butenal (CAS number 497-03-0),
- methyl butanone (CAS number: 563-80-4),
- 3-methyl-3-buten-2-one (CAS number 814-78-8),
- 3-methyl-2-pentanone (CAS number 565-61-7),
- butanal (CAS number 123-72-8),
- 5-methyl-3-hexen-2-one (CAS number 5166-53-0), and
- acetone (CAS number 67-64-1),

b) calculating the four following ratios :

- R1 = 2-methyl-2-butenal (CAS number 497-03-0) /methyl butanone (CAS number: 563-80-4),
- R2 = 3-methyl-3-buten-2-one (CAS number 814-78-8) /2-methyl-2-butenal (CAS number 497-03-0),
- R3 = 3-methyl-2-pentanone (CAS number 565-61-7) / butanal (CAS number 123-72-8), and
- R5 = 5-methyl-3-hexen-2-one (CAS number 5166-53-0) / acetone (CAS number 67-64-1),

wherein said ratios are compared to reference ratios.

In one embodiment, in the method according to the invention, step a) further comprises determining the concentration of the three following VOCs:

- 2,4 dimethyl- 3-pentanone (CAS number 565-80-0),
- dimethyl sulfide (CAS number 75-18-3), and
- 1-propanol (CAS number 71-23-8), and
  step b) further comprises calculating the two following ratios:

- R4 = 2,4 dimethyl- 3-pentanone (CAS number 565-80-0) / methyl butanone (CAS number: 563-80-4),and
- R6 = dimethyl sulfide (CAS number 75-18-3) / 1-propanol (CAS number 71-23-8).

In all embodiments, the saliva sample is stabilized prior to the analysis of VOCs content with a preservation solution comprising guanidinium thiocyanate, and/or sodium azide and/or ammonium sulfate.

The method of the invention uses the volatile fraction of saliva sample.

The method according to the invention enables the identification of a VOC signature indicative of oral cancer. This VOC signature relies on the six ratios as defined previously.

Also in certain embodiments, the invention relates to a statistical model that allows samples of subjects with oral cancer to be distinguished from those of healthy subjects. This model increases the accuracy suitable for a diagnosis method.

Another object of the present invention is to provide a kit for its use for the implementation of the method according to the invention. The kit comprises a sterile device to collect saliva sample, a collection reagent, at least one preservative solution, and optionally an electronic sensor.

## BRIEF DESCRIPTION OF THE FIGURES:

Figure 1: Factorial discriminant analysis. FDA with 54 training samples (20 healthy control and 34 cancer test participants), and 10 test validation samples (4 healthy control and 6 cancer test participants).

Figure 2: ROC curve. Receiver operating characteristic curve of oral cancer diagnosis using 6 ratios; (n = 64; n = 40 patients with oral cancer and n = 20 control subjects); the area under the ROC curve (AUROC value) was 0.999.

Figure 3: SPME-GC-MS chromatogram of a human saliva sample. A typical chromatogram of the VOCs from a saliva sample obtained from an oral cancer subject.

## DETAILED DESCRIPTION OF THE INVENTION:

In a first aspect, the present invention relates to an *in vitro* method of diagnosing oral cancer or assessing risk of developing oral cancer in a subject, comprising :

a) determining the concentration of at least the seven following volatile organic compounds (VOCs) in a saliva sample obtained from the subject:

- 2-methyl-2-butenal (CAS number 497-03-0),
- methyl butanone (CAS number: 563-80-4),
- 3-methyl-3-buten-2-one (CAS number 814-78-8),
- 3-methyl-2-pentanone (CAS number 565-61-7),
- butanal (CAS number 123-72-8),
- 5-methyl-3-hexen-2-one (CAS number 5166-53-0), and
- acetone (CAS number 67-64-1),and

b) calculating the four following ratios :

- R1 = 2-methyl-2-butenal (CAS number 497-03-0) /methyl butanone (CAS number: 563-80-4),
- R2 = 3-methyl-3-buten-2-one (CAS number 814-78-8) /2-methyl-2-butenal (CAS number 497-03-0),
- R3 = 3-methyl-2-pentanone (CAS number 565-61-7) / butanal (CAS number 123-72-8), and
- R5 = 5-methyl-3-hexen-2-one (CAS number 5166-53-0) / acetone (CAS number 67-64-1),

wherein said ratios are compared to reference ratios.

By comparing said ratios to reference ratios, it is intended that a difference in ratios for said VOCs indicates an oral cancer or a risk of developing oral cancer.

According to an alternative embodiment, the comparison between the ratios calculated in step (b) and the reference ratios constitutes a separate step in the method of the invention.

The invention thus also relates to an *in vitro* method of diagnosing oral cancer or assessing risk of developing oral cancer in a subject, comprising :

a) determining the concentration of at least the seven following volatile organic compounds (VOCs) in a saliva sample obtained from the subject:

- 2-methyl-2-butenal (CAS number 497-03-0),
- methyl butanone (CAS number: 563-80-4),
- 3-methyl-3-buten-2-one (CAS number 814-78-8),
- 3-methyl-2-pentanone (CAS number 565-61-7),
- butanal (CAS number 123-72-8),
- 5-methyl-3-hexen-2-one (CAS number 5166-53-0), and
- acetone (CAS number 67-64-1),and

b) calculating the four following ratios :

- R1 = 2-methyl-2-butenal (CAS number 497-03-0) /methyl butanone (CAS number: 563-80-4),
- R2 = 3-methyl-3-buten-2-one (CAS number 814-78-8) /2-methyl-2-butenal (CAS number 497-03-0),
- R3 = 3-methyl-2-pentanone (CAS number 565-61-7) / butanal (CAS number 123-72-8), and
- R5 = 5-methyl-3-hexen-2-one (CAS number 5166-53-0) / acetone (CAS number 67-64-1),and

c) Comparing the four calculated ratios of step b) to reference ratios, wherein a difference in ratios for said VOCs indicates an oral cancer or a risk of developing oral cancer.

The term "diagnosing" refers to assessing whether a subject suffers from the oral cancer, or not. As will be understood by those skilled in the art, such an assessment, although preferred to be, may usually not be correct for 100 percent of the investigated subjects. The term, however, requires that a statistically significant portion of subjects can be correctly assessed and, thus, diagnosed. Whether a portion is statistically significant can be determined by the person skilled in the art using various well known statistic evaluation tools, e.g., determination of confidence intervals, p-value determination, Student's t-test, Mann-Whitney test, etc.... Preferred confidence intervals are at least 50 percent, at least 60 percent, at least 70 percent, at least 80 percent, at least 90 percent or at least 95 percent. The p-values are, preferably, 0.05, 0.005, or 0.0005.

The term "oral cancer" refers to cancer of occurring in the squamous cells that line the moist, mucosal surfaces inside the head and neck (for example, inside the mouth, the nose, and the throat) or in the salivary glands. The expressions "oral cancer" and "head and neck cancer" are equivalent and can be used indifferently. Head and neck cancers include cancers in the larynx, pharynx (naso-, oro- and hypo-pharynx), throat, lips, mouth, nose, tongue, gum and salivary glands. 90% of all oral cancers are diagnosed as oral squamous cell carcinoma (OSCC) or head and neck squamous cell carcinoma (HNSCC).

According to a preferred embodiment, the oral cancer is head and neck squamous cell carcinoma (HNSCC).

The term "risk of developing" refers to the chance that a person will develop a disease during a given time. In the present case, the risk of developing oral cancer refers to the chance that a person will develop an oral cancer during his whole life. The method of the invention is especially useful for determining a patient who is at risk for developing oral cancer by assessing the volatile fraction of saliva for determining a VOC signature that is associated with oral cancer. The risk can be either a high risk of developing an oral cancer or a low risk of developing an oral cancer.

In the context of the invention, a human subject is said "to have a high risk of developing an oral cancer" when he has a risk at least superior to 60%, preferably to 70%, more preferably to 80% and even more preferably to 90% of developing an oral cancer. In other words, the human subject has a much higher probability to develop an oral cancer as compared to the normal population or to a human subject in which the VOC signature or the ratios of VOCs are different. In the context of the present invention, when a human subject has a risk superior to 97% to be developing an oral cancer, it is said that the human subject "is developing an oral cancer".

This oral cancer can be initiating or well-established. In one embodiment of the invention, the ratios of VOCs can potentially indicate the grade of the oral cancer from which the human subject is suffering.

The method according to the invention also enables to determine if a human subject has a low risk to be developing an oral cancer. In the context of the invention, the human subject has a low risk of developing an oral cancer when he has a risk of developing an oral cancer lower than 10%, preferably lower than 5% as compared with the normal population. In other words, the human subject has a chance superior to 90%, preferably 95% to be healthy, at least as far as oral cancer is concerned. In the context of the invention, when a human subject has a risk inferior to 5% of being developing an oral cancer, it is said that the human subject is not developing an oral cancer at the time of the collection of the saliva sample.

The term "subject" refers to animals and, preferably, to mammals. More preferably, the subject is a primate and, most preferably, a human. The subject, preferably, is suspected to suffer from oral cancer, i.e. it may already show some or all of the symptoms associated with the disease. The subject, preferably, is at increased risk of developing oral cancer. More preferably, such a subject being at increased risk has one or more tissue lesions, including areas of leukoplakia (an abnormal white patch of cells) and erythroplakia (an abnormal red patch of cells) and/or has a tobacco and alcohol consumption history and/or infection with high-risk types of human papillomavirus (HPV) for oral cancer. The subject may be of any age and gender.

The term "saliva sample" refers to a fluid produced by major and minor salivary glands. Major salivary glands including parotid, submandibular, and sublingual glands, are known to secrete fluid transported from serum as well as surrounding glandular tissues. This selective transportation within salivary glandular tissue is regulated by both acinar and tubular epithelial cells. Beside the secretions from salivary glands, oral mucosa, periodontium, as well as oral microflora also contribute to the final content of whole saliva. Whole saliva therefore represents a complex balance among local and systemic sources. This allows for the application of saliva in the diagnosis not only for salivary gland disorders but also for oral diseases and systemic conditions.

According to a preferred embodiment, the saliva sample used in the method according to the invention is stabilized saliva sample. By "stabilized" saliva sample is meant saliva sample in which nucleic acid species and volatile organic compounds have been preserved from degradation caused by the microflora, food and dental care products, during at least 7 days, more preferably 10 days at room temperature. The inventor previously disclosed in EP 2 321 430 B1 the composition of saliva preservation solution suitable for the implementation of the method according to the invention, wherein the preservation solution comprises a salt such as guanidinium thiocyanate, and/or ammonium sulfate, and/or sodium azide, wherein the salt is employed preferably at a concentration range between 20 mM and 6M, and more preferably at 40 mM. The addition of preservation solution to crude or raw saliva sample enables to maintain preferably at least 70%, more preferably at least 80% and even more preferably at least 90% of the amount of both VOCs and nucleic acids initially present in the spitted and collected saliva.

According to a preferred embodiment, the saliva preservation solution used in the method according to the invention comprises a salt such as guanidinium thiocyanate, and/or ammonium sulfate, and/or sodium azide, and/or monosodium citrate, and/or trisodium citrate dehydrate, wherein the range of concentration for guanidinium thiocyanate is from 1 to 6 M, preferably 2 to 4 M and the range of concentration for ammonium sulfate, sodium azide, monosodium citrate, and trisodium citrate dehydrate is from 0.05 % to 1% (w/v), preferably from 0.1 % to 0.5 % (w/v).

The term "VOC" refers to any organic compound that is volatile, i.e. that has a high vapor pressure or low boiling point, and can therefore evaporate at normal temperature and pressure. The European Union defines a VOC as "any organic compound having an initial boiling point less than or equal to 250 °C measured at a standard atmospheric pressure of 101.3 kPa. VOC can be classified according to their volatile temperature: very volatile organic compounds (VVOC) are considered volatile at a temperature ranging from <0° C to 50-100° C and volatile organic compounds (VOC) become gaseous at temperatures ranging from 50-100° C to 240-260° C.

The term "ratio" refers to the ratio of quantity or concentration of one substance to the quantity or concentration of another substance in a saliva sample. The matrix of ratios was formed for each sample as follows: the abundance of each VOC is divided by the abundance of each other VOC one by one. For instance, Ratio 1 = [mol1]/[mol2]; Ratio 2 = [mol1]/[mol3], etc., wherein mol = VOC. As an example, the 2-methyl-2-butenal to methyl butanone ratio may be simply calculated as a ratio of the abundance of 2-methyl-2-butenal to the abundance of methyl butanone.

The use of ratios is based on the hypothesis that pathological processes modify the metabolic ratios of VOCs normally generated in the healthy human body.

The 78 VOCs identified in saliva samples and used to establish the 1,767 ratios are listed in table 1 below:

**Table 1. Percentage of occurrence of compounds identified in saliva samples in the test and control groups; KI: Kovats Index; CAS = CAS number; m/z: mass-to-charge ratio used for relative-quantitation.**

| KI | Compound name | CAS number | Specific ion m/z | Control Group (%) | UADT Test Group (%) |
|---|---|---|---|---|---|
| *Volatile acids* | | | | | |
| 595 | acetic acid | 64-19-7 | 60 | 87.5 | 95.0 |
| 687 | propanoic acid | 79-09-4 | 74 | 91.7 | 95.0 |
| 775 | butanoic acid | 107-92-6 | 60 | 83.3 | 85.0 |
| 867 | pentanoic acid | 109-52-4 | 60 | 58.3 | 65.0 |
| 964 | hexanoic acid | 142-62-1 | 60 | 66.7 | 67.5 |
| 1158 | octanoic acid | 124-07-2 | 60 | 45.8 | 50.0 |
| 1262 | nonanoic acid | 112-05-0 | 60 | 79.2 | 82.5 |
| 746 | 2-methylpropanoic | 79-31-2 | 73 | 70.8 | 82.5 |
| 830 | 3-methylbutanoic acid | 503-74-2 | 60 | 75.0 | 77.5 |
| 843 | 2-methylbutanoic acid | 116-53-0 | 74 | 75.0 | 80.0 |
| 938 | 4-methylpentanoic acid | 646-07-1 | 74 | 37.5 | 52.5 |
| *Aldehydes* | | | | | |
| 389 | ethanal | 75-07-0 | 43 | 100.0 | 100.0 |
| 592 | butanal | 123-78-8 | 72 | 100.0 | 100.0 |
| 698 | pentanal | 110-62-3 | 58 | 100.0 | 100.0 |
| 799 | hexanal | 66-25-1 | 56 | 100.0 | 100.0 |
| 1003 | octanal | 124-13-0 | 84 | 91.7 | 100.0 |
| 1104 | nonanal | 124-19-6 | 57 | 91.7 | 95.0 |
| 551 | 2-methylpropanal | 78-84-2 | 72 | 95.8 | 92.5 |
| 651 | 3-methylbutanal | 590-86-3 | 58 | 41.7 | 75.0 |
| 905 | 2-ethylhexanal | 123-05-7 | 72 | 95.8 | 92.5 |
| 559 | 2-methyl,2-propenal | 78-85-3 | 70 | 75.0 | 85.0 |
| 742 | 2-methyl,2-butenal | 497-03-0 | 84 | 100.0 | 100.0 |
| 965 | benzaldehyde | 100-52-7 | 77 | 83.3 | 87.5 |
| 1091 | 2-methylbenzaldehyde | 529-20-4 | 119 | 62.5 | 57.5 |
| *Alkanes and alkenes* | | | | | |
| 600 | hexane | 110-54-3 | 86 | 100.0 | 100.0 |
| 663 | cyclohexane | 110-82-7 | 84 | 100.0 | 100.0 |
| 726 | methylcyclohexane | 108-82-7 | 83 | 75.0 | 77.5 |
| 628 | methylcyclopentane | 96-37-7 | 56 | 100.0 | 100.0 |
| 512 | 1,2-dimethylcyclopropane | 2452-99-5 | 55 | 100.0 | 100.0 |
| 516 | 2-methyl,2-butene | 513-35-9 | 70 | 100.0 | 95.0 |
| *Benzyl and phenyl hydrocarbons (including alcohol)* | | | | | |
| 978 | phenol | 108-95-2 | 94 | 83.3 | 87.5 |
| 1073 | p-cresol | 106-44-5 | 107 | 58.3 | 62.5 |

(continued)

| Furans | | | | | |
|---|---|---|---|---|---|
| 832 | 2-furfural | 98-01-1 | 96 | 54.2 | 80.0 |
| 1133 | 2-methyl-5-propionylfuran | 10599-69-6 | 109 | 66.7 | 80.0 |
| Nitrogen containing volatiles | | | | | |
| 985 | aniline | 62-53-3 | 93 | 58.3 | 57.5 |
| 753 | 1H-pyrrole | 109-97-7 | 67 | 50.0 | 40.0 |
| 1314 | indole | 120-72-9 | 117 | 66.7 | 62.5 |
| 1405 | skatole | 83-34-1 | 130 | 16.7 | 32.5 |
| Alcohols | | | | | |
| 492 | 2-propanol | 67-63-0 | 45 | 100.0 | 95.0 |
| 550 | 1-propanol | 71-23-8 | 59 | 100.0 | 100.0 |
| 792 | 3-hexanol | 623-37-0 | 59 | 70.8 | 72.5 |
| 867 | 1-hexanol | 111-27-3 | 56 | 83.3 | 80.0 |
| 519 | 2-methyl-2-propanol, | 75-65-0 | 59 | 100.0 | 100.0 |
| 639 | 2-methyl-2-butanol | 75-85-4 | 59 | 100.0 | 100.0 |
| 729 | 2-methyl-2-pentanol | 590-36-3 | 59 | 95.8 | 97.5 |
| 732 | 3-methyl-1-butanol | 123-51-3 | 55 | 95.8 | 95.0 |
| 788 | 2,3-dimethyl-2-pentanol | 4911-70-0 | 59 | 100.0 | 100.0 |
| 857 | 2,5-dimethyl-2-hexanol | 3730-60-7 | 59 | 100.0 | 100.0 |
| 1029 | 2-ethyl-1-hexanol | 104-76-7 | 57 | 87.5 | 80.0 |
| Ketones | | | | | |
| 488 | acetone | 67-64-1 | 58 | 100.0 | 100.0 |
| 596 | 2-butanone | 78-93-3 | 72 | 100.0 | 100.0 |
| 685 | 2-pentanone | 107-87-9 | 86 | 100.0 | 100.0 |
| 787 | 3-hexanone | 589-38-8 | 100 | 100.0 | 100.0 |
| 788 | 2-hexanone | 591-78-6 | 58 | 100.0 | 100.0 |
| 889 | 2-heptanone | 110-43-0 | 58 | 100.0 | 100.0 |
| 991 | 2-octanone | 111-13-7 | 58 | 100.0 | 100.0 |
| 657 | methyl butanone | 563-80-4 | 86 | 100.0 | 100.0 |
| 711 | 3,3-dimethyl-2-butanone | 75-97-8 | 100 | 91.7 | 87.5 |
| 738 | 4-methyl-2-pentanone | 108-10-1 | 58 | 100.0 | 100.0 |
| 750 | 2-methyl-3-pentanone | 565-69-5 | 57 | 100.0 | 97.5 |
| 752 | 3-methyl-2-pentanone | 565-61-7 | 57 | 100.0 | 100.0 |
| 781 | 4,4-dimethyl-2-pentanone | 590-50-1 | 58 | 100.0 | 100.0 |
| 791 | 2,4 dimethyl-3-penta none | 565-80-0 | 71 | 100.0 | 100.0 |
| 1075 | acetophenone | 98-86-2 | 105 | 79.2 | 77.5 |
| 896 | cyclohexanone | 108-94-1 | 55 | 100.0 | 100.0 |
| 587 | 2,3-butanedione | 431-03-8 | 86 | 100.0 | 100.0 |
| 688 | 2,3-pentanedione | 600-14-6 | 100 | 91.7 | 95.0 |

(continued)

| Ketones | | | | | |
|---|---|---|---|---|---|
| 826 | 3-methyl-2,4-pentanedione | 815-57-6 | 72 | 100.0 | 100.0 |
| 671 | 3-methyl-3-buten-2-one | 814-78-8 | 84 | 100.0 | 100.0 |
| 824 | 5-methyl-3-hexen-2-one | 5166-53-0 | 97 | 100.0 | 97.5 |
| 839 | 3-methyl-3-penten-2-one | 565-62-8 | 98 | 87.5 | 92.5 |
| 960 | 3,4-dimethyl-3-hexen-2-one | 1635-02-5 | 55 | 79.2 | 80.0 |
| 710 | 3-hydroxy-2-butanone | 513-86-0 | 88 | 83.3 | 77.5 |
| Sulfur-containing volatiles | | | | | |
| 417 | methanethiol | 74-93-1 | 47 | 37.5 | 52.5 |
| 518 | dimethylsulfide | 75-18-3 | 62 | 62.5 | 65.0 |
| 747 | dimethyldisulfide | 624-92-0 | 94 | 66.7 | 67.5 |
| 913 | dimethylsulfone | 67-71-0 | 79 | 95.8 | 92.5 |
| 786 | 3-methylthiophene | 616-44-4 | 97 | 100.0 | 100.0 |

COVs listed in table 1 are volatile acids, aldehydes, alkanes and alkenes, benzyl and phenyl hydrocarbons, furans, nitrogen containing volatiles, alcohols, ketones, sulfur-containing volatiles and other volatile compounds.

In all embodiments, the COVs identified and used in the method according to the invention are selected in the group comprising aldehydes and ketones. More particularly, the COVs 2-methyl-2-butenal (CAS number 497-03-0), methyl butanone (CAS number 563-80-4), 3-methyl-3-buten-2-one (CAS number 814-78-8), 3-methyl-2-pentanone (CAS number 565-61-7), 5-methyl-3-hexen-2-one (CAS number 5166-53-0) and acetone (CAS number 67-64-1) are identified and quantified for the calculation of the four ratios R1, R2, R3 and R5.

The term "reference" refers to values of characteristic features of each of the VOC which can be correlated to an oral cancer, i.e. the presence or absence of the oral cancer or oral cancer grade. Preferably, a reference is a threshold value (e.g., an amount or ratio of amounts) for a VOC whereby values found in a sample to be investigated which are higher than or essentially identical to the threshold are indicative for the presence of an oral cancer while those being lower are indicative for the absence of the oral cancer. It will be understood that also preferably, a reference may be a threshold value for a VOC whereby values found in a sample to be investigated which are lower or identical than the threshold are indicative for the presence of an oral cancer while those being higher are indicative for the absence of the oral cancer.

In accordance with the aforementioned method of the present invention, a reference is, preferably, a reference obtained from a sample from a subject or group of subjects known to suffer from oral cancer. In such a case, a value for the at least four ratios of VOCs found in the test sample being essentially identical is indicative for the presence of the disease. Moreover, the reference, also preferably, could be from a subject or group of subjects known not to suffer from oral cancer, preferably, an apparently healthy subject. In such a case, a value for the at least four ratios of VOCs found in the test sample being altered with respect to the reference is indicative for the presence of the oral cancer. The group of subjects referred to before shall comprise a plurality of subjects, preferably, at least 5, 10, 50, 100, 1,000 or 10,000 subjects. It is to be understood that the subject to be diagnosed by the method of the present invention and the subjects used as reference are of the same species.

The term "comparing" refers to determining whether the determined value of a VOC or a ratio of VOC is essentially identical to a reference or differs there from. Preferably, a value or ratio for a VOC is deemed to differ from a reference if the observed difference is statistical significant which can be determined by statistical techniques referred to elsewhere in this description. If the difference is not statistically significant, the VOC value or ratio and the reference are essentially identical. Based on the comparison referred to above, a subject can be assessed to suffer from the oral cancer, or not.

The comparison is, preferably, assisted by automation. For example, a suitable computer program comprising algorithms for the comparison of two different data sets (i.e., data sets comprising the values of the characteristic feature(s)) may be used. Such computer programs and algorithms are well known in the art. Notwithstanding the above, a comparison can also be carried out manually.

According to a preferred embodiment, the VOCs are detected in the volatile fraction of saliva sample. In the context of the invention, the "volatile fraction" is recovered from the heating of a crude saliva sample. Preferably, said volatile fraction is extracted from crude saliva sample after thawed for one hour at ambient temperature and then by heating said saliva sample for at least 2 minutes, preferably 5 minutes and more preferably 10 minutes at a temperature comprised between 30°C and 50°C, preferably 37°C. VOCs are then extracted using a preconditioned PDMS (polydimethylsiloxane) /CAR (CARBOXEN®) SPME (solide-phase microextraction) fiber exposed to the headspace above the heated sample for 30 minutes.

According to a preferred embodiment, the VOCs are detected using mass spectrometry.

Therefore, in one embodiment of the present invention, the volatile organic compounds are extracted with a PDMS/CAR SPME fiber during at least, preferably 20 minutes, and even more preferably 30 minutes from a saliva sample that is simultaneously heated at a temperature comprised between 30°C and 50°C, and preferably at about 37°C. The desorption temperature of the fiber is comprised between 250°C and 300°C, and is preferably of about 280°C.

Solid-phase microextraction (SPME) is a patented sample preparation technique based on the adsorption of analytes directly from an aqueous sample onto a coated, fused-silica fiber. This sampling technique is fast, easy to use and eliminates the use of organic solvents.

In this technology, the PDMS/CAR SPME fibers are often used for detecting trace level of volatile organic compounds, and are therefore well-known from the man skilled in the art.

According to a preferred embodiment, the detection of volatile organic compounds is performed by using a chromatograph in gas phase coupled to a mass spectrometer (GC-MS).

According to a preferred embodiment, the mass spectrometry used to detect VOCs is SPME-GC-MS.

In the context of the invention, a VOC is "detected" when its concentration or ratio of VOCs as mentioned above is at least superior to 1.5 fold the mean concentration or ratio of said compound in the healthy population. By "healthy population" is meant a group of healthy subjects.

By applying the method of the invention, some biochemical compounds were shown to be highly overexpressed in the volatile fraction of saliva of human subjects suffering from oral cancer and were therefore found to be acute and sensitive diagnostic and/or prognostic tool of oral cancer. Importantly, none of these compounds can be detected in the fluid fraction of saliva, highlighting the necessity to study the volatile fraction of saliva in this case.

VOCs are identified with their Kovats indices, m/z relative quantification levels, and their percentages of occurrence in both groups of healthy subjects and oral cancer suffering patients. As used herein, the term "m/z value" refers to the mass-to-charge ratio of an ion produced in an ionization source, as measured by a mass spectrometer. This term can also be extended to other analytical methods, such as, but not limited to ion mobility, that analyze ions produced in an ionization source and can refer to the collision cross section, drift time, or compensation voltage (CV) of an ion.

According to a preferred embodiment, in the method according to the invention step a) further comprises determining the concentration of the three following VOCs:

- 2,4 dimethyl- 3-pentanone (CAS number 565-80-0),

- dimethyl sulfide (CAS number 75-18-3), and

- 1-propanol (CAS number 71-23-8), and

step b) further comprises calculating the two following ratios:

- R4 = 2,4 dimethyl- 3-pentanone (CAS number 565-80-0) / methyl butanone (CAS number: 563-80-4),and

- R6 = dimethyl sulfide (CAS number 75-18-3) / 1-propanol (CAS number 71-23-8).

Thus, the method according to the invention allows the identification and quantification of alcohols, ketones and sulfur-containing volatiles. More particularly, the COVs 2,4 dimethyl- 3-pentanone (CAS number 565-80-0), methyl butanone (CAS number: 563-80-4), dimethyl sulfide (CAS number 75-18-3) and 1-propanol (CAS number 71-23-8) are identified and quantified for the calculation of the two ratios R4 and R6.

According to this preferred embodiment, the invention relates to an *in vitro* method of diagnosing oral cancer or assessing risk of developing oral cancer in a subject, comprising :

a) determining the concentration of at least the ten following volatile organic compounds (VOCs) in a saliva sample obtained from the subject:

- 2-methyl-2-butenal (CAS number 497-03-0),
- methyl butanone (CAS number: 563-80-4),
- 3-methyl-3-buten-2-one (CAS number 814-78-8),
- 3-methyl-2-pentanone (CAS number 565-61-7),
- butanal (CAS number 123-72-8),
- 5-methyl-3-hexen-2-one (CAS number 5166-53-0),
- acetone (CAS number 67-64-1),
- 2,4 dimethyl- 3-pentanone (CAS number 565-80-0),
- dimethyl sulfide (CAS number 75-18-3), and
- 1-propanol (CAS number 71-23-8), and

b) calculating the six following ratios :

- R1 = 2-methyl-2-butenal (CAS number 497-03-0) /methyl butanone (CAS number: 563-80-4),
- R2 = 3-methyl-3-buten-2-one (CAS number 814-78-8) /2-methyl-2-butenal (CAS number 497-03-0),
- R3 = 3-methyl-2-pentanone (CAS number 565-61-7) / butanal (CAS number 123-72-8),
- R5 = 5-methyl-3-hexen-2-one (CAS number 5166-53-0) / acetone (CAS number 67-64-1),
- R4 = 2,4 dimethyl- 3-pentanone (CAS number 565-80-0) / methyl butanone (CAS number: 563-80-4),and
- R6 = dimethyl sulfide (CAS number 75-18-3) / 1-propanol (CAS number 71-23-8),

wherein said ratios are compared to reference ratios.

According to an alternative embodiment, the comparison between the ratios calculated in step (b) and the reference ratios constitutes a separate step in the method of the invention.

The invention thus also relates to an *in vitro* method of diagnosing oral cancer or assessing risk of developing oral cancer in a subject, comprising :

a) determining the concentration of at least the ten following volatile organic compounds (VOCs) in a saliva sample obtained from the subject:

- 2-methyl-2-butenal (CAS number 497-03-0),
- methyl butanone (CAS number: 563-80-4),
- 3-methyl-3-buten-2-one (CAS number 814-78-8),
- 3-methyl-2-pentanone (CAS number 565-61-7),
- butanal (CAS number 123-72-8),
- 5-methyl-3-hexen-2-one (CAS number 5166-53-0),
- acetone (CAS number 67-64-1),
- 2,4 dimethyl- 3-pentanone (CAS number 565-80-0),
- dimethyl sulfide (CAS number 75-18-3), and
- 1-propanol (CAS number 71-23-8),

b) calculating the six following ratios :

- R1 = 2-methyl-2-butenal (CAS number 497-03-0) /methyl butanone (CAS number: 563-80-4),
- R2 = 3-methyl-3-buten-2-one (CAS number 814-78-8) /2-methyl-2-butenal (CAS number 497-03-0),
- R3 = 3-methyl-2-pentanone (CAS number 565-61-7) / butanal (CAS number 123-72-8),
- R5 = 5-methyl-3-hexen-2-one (CAS number 5166-53-0) / acetone (CAS number 67-64-1),
- R4 = 2,4 dimethyl- 3-pentanone (CAS number 565-80-0) / methyl butanone (CAS number: 563-80-4),and
- R6 = dimethyl sulfide (CAS number 75-18-3) / 1-propanol (CAS number 71-23-8), and

c) Comparing the six calculated ratios of step b) to reference ratios, wherein a difference in ratios for said VOCs indicates an oral cancer or a risk of developing oral cancer.

The calculation of the ratios R1, R2, R3, R4, R5 and R6 enables the determination of a VOC signature specific for oral cancer. By "VOC signature" is intended a unique combination or cluster of VOCs found in saliva that is a sign of oral cancer. In other words, VOC signature refers to a specific signature of a set (i.e., two or more, three or more, four or more, and so on) of identified VOCs that are specific to oral cancer. The VOC signature for a specific sample from a subject may include the presence/absence/quantity/ratio, etc. regarding a set of identified VOC compounds. The VOC signature is identification and calculation of the concentrations/amounts/ratios of various VOCs present in the sample.

VOC signature molecule may be used as a diagnostic and/or prognostic tool of oral cancer. VOC signature allows discrimination between healthy subject and patient having oral cancer. Large scale analysis of VOCs in saliva sample can provide identification and quantitative VOC information allowing for the generation of a VOC signature, each indicative of oral cancer. The method according to the invention enables quantitatively measuring the amount of each VOC alone or in combination in saliva samples. In addition, this method identifies optimized subsets of VOC that, when used in ratios, are highly sensitive and specific for oral cancer.

Assessing whether specific VOCs used as biomarkers for oral cancer has clinical validity requires estimation of sensitivity and specificity, which can be summarized with the receiver operator characteristic (ROC) curve. By plotting the true positive rates (sensitivity) versus the false positive rates (1-specificity) across all possible thresholds, ROC curve reflects the relative trade-off between true and false positive rates. The area under the ROC curve (AUC) measures the distance between the distributions of diseased and non-diseased populations and is frequently used as a global measure for the accuracy of the diagnostic test.

The generation of ROC curves and analysis of a population of samples is used to establish the cutoff value used to distinguish between different subject sub-groups. For example, the cutoff value may distinguish between subjects being at high risk or low risk of developing oral cancer. In some aspects, the cutoff value may distinguish between subjects that have oral cancer from subjects that do not have oral cancer.

In order to improve diagnostic accuracy, i.e. to maximize the separation among the groups of healthy subjects and oral cancer suffering subjects, the inventor developed a linear combination (LC) based on the identification and quantification of the specific VOCs ratios R1, R2, R3, R4, R5 and R6. This LC aimed at maximizing the area under the ROC curve (AUC).

According to a preferred embodiment, the method according to the invention further comprises a step of calculating a linear combination (LC) based on the six ratios of VOCs mentioned here above. This linear combination has the following formula:

$$LC = 2.51 - A \times (R1) + B \times (R2) - C \times (R3) - D \times (R4) + E \times (R5) + F \times (R6),$$

wherein A, B, C, D, E and F are constants. The values attributed to these constants are respectively : A = 4.88, B = 1.21, C = 1.51, D = 4.33, E = 70.49 and F = 21.01.

In a preferred embodiment, oral cancer or increased risk of developing oral cancer is diagnosed if LC is lower than or equal to +1.5 (LC ≤ +1.5).

If LC is higher than +1.5 (LC>+1.5), the sample is classified healthy.

If LC is lower than or equal to -0.5 (LC≤-0.5), the sample is classified as high risk of having or developing an oral cancer.

The optimal linear combination implemented in the method according to the invention enables diagnosing or assessing the risk of developing oral cancer with high specificity and sensitivity.

"Sensitivity" in the sense of the invention refers to the assay result of true positives in the analysis of oral cancer. Preferably the sensitivity in the analysis according to the invention is set to 90 percent to 100 percent, preferably 92 to 95 percent, and most preferably 100 percent (i.e. no false negative diagnoses).

"Specificity" in the sense of the invention is the so-called true negative rate in an assay to diagnose oral cancer. The specificity is preferably targeted to be at least 90 percent and preferably higher, e.g. 92 percent, 95 percent, 98 percent, 99 percent.

According to a preferred embodiment, the diagnosis or assessing the risk of oral cancer has a specificity of at least 95% and a sensitivity of at least 92%.

The present invention also relates to a kit for the implementation of the method according to the invention.

In a preferred embodiment, the kit comprises a sterile device to collect saliva sample, a collection reagent, at least one preservation solution, and optionally, an electronic sensor.

The collect reagent may be a dilution buffer which is preferably a citrate buffer or a tartrazine solution.

The kit comprises a preservation solution, which is preferably a buffer comprising a salt capable of reducing the vapor tension of volatile compounds without allowing the degradation of said compounds. This salt is preferably a salt such as guanidinium thiocyanate, and/or ammonium sulfate and/or sodium azide, and/or monosodium citrate, and/or trisodium citrate dehydrate.

According to a preferred embodiment, the preservation solution of the comprises a salt such as guanidinium thiocyanate, and/or ammonium sulfate, and/or sodium azide, and/or monosodium citrate, and/or trisodium citrate dehydrate, wherein the range of concentration for guanidinium thiocyanate is from 1 to 6 M, preferably 2 to 4 M and the range of concentration for ammonium sulfate, sodium azide, monosodium citrate, and trisodium citrate dehydrate is from 0.05 % to 1% (w/v), preferably from 0.1 % to 0.5 % (w/v).

In one embodiment of the present invention, the preservation solution is provided in a dry format in a sterile plastic tube under vacuum, which can draw up the saliva associated with the dilution buffer.

In the context of the invention, the device used to detect the organic compounds in the collected volatile fraction of saliva is an electronic sensor, for example electronic noses, JPL electronic noses, FET-type Bioelectronic noses, alpha mos. These technologies are now widely used and therefore known from the man skilled in the art.

Using specific electronic sensors for the identification of the targeted volatile compounds in a specific platform (electronic nose), the invention enables the user to perform a specific analysis platform or a point of care analysis usable in physician and dentists offices.

In one embodiment of the invention the control molecular markers are chosen among: 1-bromobutane, 1-bromobenzene and 1,4-dibromobenzene.

The present invention also relates to the use of a kit for the implementation of the method according to the invention, wherein said kit comprises a sterile device to collect a saliva sample, a collection reagent, at least one preservation solution, and optionally, an electronic sensor.

The following examples are provided in order to demonstrate and further illustrate certain preferred embodiments and aspects of the present invention and are not to be construed as limiting the scope thereof.

## EXAMPLES

### 1. Study population

The data analyzed in this study relate to 40 subjects who were diagnosed with a squamous cell carcinoma of head and neck (HNSCC). The study group included 8 females and 32 males, mean age 63.3 (range 37-100), who were compared to a control group constituted with 24 healthy participants, 10 females and 14 males, mean age 41.4 (range 23-58). Among the subjects, 2 were edentulous and 9 had clinically-determined poor oral health (periodontal diseases, dental caries, etc.). Twenty four were regular users of tobacco products and consumers of alcoholic beverages, although 3 subjects had terminated tobacco before the start of the study. Detailed data of study patients and control groups are summarized in Table 2.

**Table 2. Description of the study population; *Female; M: Male**

|  | Patients group | Control group |
|---|---|---|
| Number | 40 | 24 |
| Mean age (years) | 63.3 | 41.4 |
| Gender (F/M)* | 8F/32M | 10F/14M |
| Tabacco consummers | 26 | 12 |
| Alcohol consummers | 27 | 2 |
| Poor oral health | 9 | 1 |
| *F: female and M: male | | |

### 2. Saliva collection and sample preparation

Saliva was collected using 4.0 ml of a tartrazine solution before stabilization with 4 mg of sodium azide and 1.9 mg/ml of ammonium sulfate as preservatives for metabolites and nucleic acids at ambient temperature for a period of up to 7 days. To avoid external VOCs contamination or their salivary dilution, study participants were instructed to refrain from smoking, tooth-brushing or mouthwash use, food or fluid ingestion, or chewing gum for a period of at least 1 hour prior the saliva collection process.

### 3. Sample controls

Three parameters were controlled before the VOCs analysis: sample volume, raw saliva quantity and total bacterial flora. The minimum sample volume required to allow the performance of all analyses was 3.5 ml. The amount of saliva was assayed by spectrometry; the sample had to contain at least 28% of stimulated saliva. Quantitative real-time PCR was performed to determine total bacterial counts (TBCs). Total nucleic acid extraction was performed from 250 $\mu$l of saliva with the NUCLESPIN kit provided by MACHEREY NAGEL (Germany). Quantitative real-time PCR assays were performed in a volume of 10 $\mu$l composed of 1× QUANTIFAST® SYBR® Green PCR (Qiagen, Germany),

2 $\mu$l of DNA extract, and 1 $\mu$M of each primer. The bacterial PCR primers used in this study were manufactured by METABION GmbH (MARTINSRIED, Germany). Bacterial primers were derived from universal ribosomal 16S sequences (22) adapted to real-time PCR conditions. Assays were conducted on the ROTOR-GENE® Q thermal cycling system (QIAGEN, Germany) with the following program: 95°C for 5 min., followed by 40 cycles of 10 s at 95°C, 10 s at 60°C, and 35 s at 72°C. A final melt curve analysis (70°C to 95°C in 1°C steps for 5 s increments) was then completed. Fluorescence signals were measured every cycle at the end of the extension step and continuously during the melt curve analysis. Resulting data were analyzed using Rotor-Gene® Q Series software (QIAGEN, Germany). Serial dilutions of bacterial standard DNA from E.coli were employed in each reaction as external standards for absolute quantification of total bacterial levels. Standard bacterial strains used for standard nucleic acid production were obtained from DSMZ (Germany). It was ensured that a sample of acceptable quality had to have a total amount of flora $\geq 5.11$ CFU/mL of saliva.

## 4. Sample preparation

For VOCs analyses, 1.00 mL aliquots of saliva sample were placed in 10 mL vials (VWR) with 10 $\mu$l of a solution of external standards up to levels of 1.00 ppm (1-bromobutane, 1-bromobenzene and 1,4-dibromobenzene, Sigma-Aldrich). The vials containing the samples were then sealed with crimp cap Silicone/PTFE (VWR), and then stored at -80°C prior to analyses. An external standard was then injected to allow the validation process via the determination of relative abundances.

## 5. SPME-GC-MS analysis

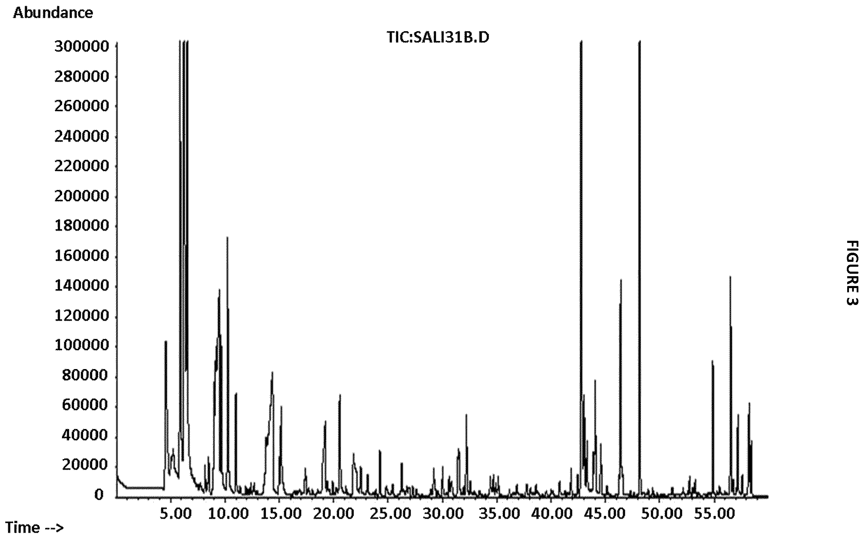

An analytical method was developed to optimize the experimental conditions, and a PDMS/CAR, 75 $\mu$m (SUPELCO, Bellefonte, Pa.) fiber was chosen to trap VOCs in this study. Before analysis, stored samples were thawed for 1.0 hr. at ambient temperature and were then placed in a water bath at 37°C for 10 min. VOCs were then extracted using a preconditioned PDMS/CAR SPME fiber exposed to the headspace above the heated sample for 30 min. The fiber was then immediately transferred to the heated injection port (280°C) of the gas chromatograph (6890N, AGILENT TECHNOLOGIES, Palo Alto, CA, USA) for 2 min. in a splitless mode. The GC-MS system was fitted with a SPB-5 column (60 m x 0.32 mm ID x 1 $\mu$m, SUPELCO, Bellefonte, Pa) and linked with a mass spectrometer (5973 inert, AGILENT TECHNOLOGIES, Palo Alto, CA, USA). The GC oven temperature was maintained at 40°C for 5 min. after the injection, and then heated at a rate of 3°C/min. to 230°C, and then held at this temperature for 2 min., giving a total run time of 70.33 minutes. Pure helium (Helium 5.5, Messer, France) was used as the carrier gas at a constant linear debit of 1.00 ml/min. A typical chromatogram with peaks representing individual compounds is shown in Figure 3.

The operating temperatures of the MS were 280°C for the transfer line, 150°C for the quadrupole, and 230°C for the ionization source. Mass spectra were measured at an electron impact at 70 eV. The MS was operated in an electron ionization scan mode, with a mass range of 33-250 mass-to-charge (m/z) ratio values.

VOCs identification was achieved with a mass spectral bank (Wiley 275K), and with Kovats indices (KI) calculations. Relative quantifications of molecules was performed by SIM mode ion extraction to avoid errors linked to molecular co-elutions. Peak areas corresponding to the m/z values of each molecule were estimated using the Areas Arbitrary Unit (AAU).

## 6. Statistical analysis

The major motivation for using ratios rather than the measured value of each compound was to perform internal calibrations for each sample.

For each sample, 78 molecules were investigated (see Table 1), and their abundances computed. From these 78 molecules, 1,767 molecular concentration ratios were obtained.

The matrix of ratios was formed as follows: for each sample, the abundance of each molecule was divided by the abundance of each other one by one: either Ratio 1 = [Mol1] / [Mol2]; Ratio 2 = [Mol1] / [Mol3], etc.

ANOVA was performed on these 1,767 ratios according to the factor "tumor", and was used to select significant ratios ($p < 0.05$). Three hundred and seventy three ratios were selected, and a false discovery rate (FDA) consideration according to the factor "tumor" was performed on these 373 ratios on 54 samples (20 controls and 34 UADT patients) for the training and 10 samples (4 controls and 6 UADT patients) for test set verifications. UADT means Upper Aerodigestive Tract. ANOVA and FDA were confirmed with STATISTICA software (STATSOFT FRANCE, version 8.0, 2007).

**7. Results**

**• Identification of volatile organic compounds**

One hundred and seven VOCs were identified in saliva samples, and these are presented in Table 3 with their Kovats indices, m/z relative quantification levels, and their percentages of occurrence in the two groups of participants recruited to the study.

**Table 3. Percentage of occurrence of compounds identified in saliva samples in the test and control groups; KI: Kovats Index; CAS = CAS number; m/z: mass-to-charge ratio used for relative-quantitation.**

| KI | Compound name | CAS number | Specific ion m/z | Control Group (%) | UADT Test Group (%) |
|---|---|---|---|---|---|
| *Volatile acids* | | | | | |
| 595 | acetic acid | 64-19-7 | 60 | 87.5 | 95.0 |
| 687 | propanoic acid | 79-09-4 | 74 | 91.7 | 95.0 |
| 775 | butanoic acid | 107-92-6 | 60 | 83.3 | 85.0 |
| 867 | pentanoic acid | 109-52-4 | 60 | 58.3 | 65.0 |
| 964 | hexanoic acid | 142-62-1 | 60 | 66.7 | 67.5 |
| 1158 | octanoic acid | 124-07-2 | 60 | 45.8 | 50.0 |
| 1262 | nonanoic acid | 112-05-0 | 60 | 79.2 | 82.5 |
| 746 | 2-methylpropanoic | 79-31-2 | 73 | 70.8 | 82.5 |
| 830 | 3-methylbutanoic acid | 503-74-2 | 60 | 75.0 | 77.5 |
| 843 | 2-methylbutanoic acid | 116-53-0 | 74 | 75.0 | 80.0 |
| 938 | 4-methylpentanoic acid | 646-07-1 | 74 | 37.5 | 52.5 |
| *Aldehydes* | | | | | |
| 389 | ethanal | 75-07-0 | 43 | 100.0 | 100.0 |
| 592 | butanal | 123-78-8 | 72 | 100.0 | 100.0 |
| 698 | pentanal | 110-62-3 | 58 | 100.0 | 100.0 |
| 799 | hexanal | 66-25-1 | 56 | 100.0 | 100.0 |
| 1003 | octanal | 124-13-0 | 84 | 91.7 | 100.0 |
| 1104 | nonanal | 124-19-6 | 57 | 91.7 | 95.0 |
| 551 | 2-methylpropanal | 78-84-2 | 72 | 95.8 | 92.5 |
| 651 | 3-methylbutanal | 590-86-3 | 58 | 41.7 | 75.0 |
| 905 | 2-ethylhexanal | 123-05-7 | 72 | 95.8 | 92.5 |
| 559 | 2-methyl,2-propenal | 78-85-3 | 70 | 75.0 | 85.0 |
| 742 | 2-methyl,2-butenal | 497-03-0 | 84 | 100.0 | 100.0 |
| 965 | benzaldehyde | 100-52-7 | 77 | 83.3 | 87.5 |
| 1091 | 2-methylbenzaldehyde | 529-20-4 | 119 | 62.5 | 57.5 |
| *Alkanes and alkenes* | | | | | |
| 600 | hexane | 110-54-3 | 86 | 100.0 | 100.0 |
| 560 | 2-methylpentane | 107-83-5 | 71 | 100.0 | 100.0 |
| 580 | 3-methylpentane | 96-14-0 | 57 | 100.0 | 100.0 |
| 663 | cyclohexane | 110-82-7 | 84 | 100.0 | 100.0 |

(continued)

| Alkanes and alkenes | | | | | |
|---|---|---|---|---|---|
| 726 | methylcyclohexane | 108-82-7 | 83 | 75.0 | 77.5 |
| 628 | methylcyclopentane | 96-37-7 | 56 | 100.0 | 100.0 |
| 512 | 1,2-dimethylcyclopropane | 2452-99-5 | 55 | 100.0 | 100.0 |
| 397 | 2-methylpropene | 763-29-1 | 56 | 100.0 | 100.0 |
| 516 | 2-methyl,2-butene | 513-35-9 | 70 | 100.0 | 95.0 |
| Benzyl and phenyl hydrocarbons (including alcohol) | | | | | |
| 662 | benzene | 71-43-2 | 78 | 100.0 | 100.0 |
| 770 | toluene | 108-88-3 | 91 | 100.0 | 100.0 |
| 866 | ethylbenzene | 100-41-4 | 91 | 41.7 | 67.5 |
| 875 | m-xylene | 108-38-3 | 91 | 100.0 | 100.0 |
| 1082 | benzene, (2-methylpropenyl) | 768-49-0 | 117 | 87.5 | 87.5 |
| 978 | phenol | 108-95-2 | 94 | 83.3 | 87.5 |
| 1073 | p-cresol | 106-44-5 | 107 | 58.3 | 62.5 |
| Furans | | | | | |
| 603 | 2-methylfuran | 534-22-5 | 82 | 100.0 | 90.0 |
| 613 | 3-methylfuran | 930-27-8 | 82 | 100.0 | 97.5 |
| 704 | 2-ethylfuran | 3208-16-0 | 81 | 100.0 | 97.5 |
| 832 | 2-furfural | 98-01-1 | 96 | 54.2 | 80.0 |
| 1133 | 2-methyl-5-propionylfuran | 10599-69-6 | 109 | 66.7 | 80.0 |
| Nitrogen containing volatiles | | | | | |
| 519 | acrylonitrile | 107-13-1 | 53 | 87.5 | 55.0 |
| 577 | propanenitrile | 107-12-0 | 54 | 91.7 | 95.0 |
| 667 | butanenitrile | 109-74-0 | 41 | 100.0 | 100.0 |
| 774 | pentanenitrile | 110-59-8 | 54 | 100.0 | 100.0 |
| 877 | hexanenitrile | 628-73-9 | 54 | 91.7 | 100.0 |
| 979 | heptanenitrile | 629-08-3 | 82 | 91.7 | 92.5 |
| 645 | 3-butenenitrile | 109-75-1 | 67 | 54.2 | 55.0 |
| 985 | aniline | 62-53-3 | 93 | 58.3 | 57.5 |
| 991 | benzonitrile | 100-47-0 | 103 | 79.2 | 77.5 |
| 555 | ethylisocyanate | 109-90-0 | 56 | 100.0 | 95.0 |
| 964 | benzene isocyanato | 103-71-9 | 119 | 95.8 | 97.5 |
| 753 | 1H-pyrrole | 109-97-7 | 67 | 50.0 | 40.0 |
| 1314 | indole | 120-72-9 | 117 | 66.7 | 62.5 |
| 1405 | skatole | 83-34-1 | 130 | 16.7 | 32.5 |
| Alcohols | | | | | |
| 492 | 2-propanol | 67-63-0 | 45 | 100.0 | 95.0 |
| 550 | 1-propanol | 71-23-8 | 59 | 100.0 | 100.0 |

(continued)

| Alcohols | | | | | |
|---|---|---|---|---|---|
| 792 | 3-hexanol | 623-37-0 | 59 | 70.8 | 72.5 |
| 867 | 1-hexanol | 111-27-3 | 56 | 83.3 | 80.0 |
| 519 | 2-methyl-2-propanol, | 75-65-0 | 59 | 100.0 | 100.0 |
| 639 | 2-methyl-2-butanol | 75-85-4 | 59 | 100.0 | 100.0 |
| 729 | 2-methyl-2-pentanol | 590-36-3 | 59 | 95.8 | 97.5 |
| 732 | 3-methyl-1-butanol | 123-51-3 | 55 | 95.8 | 95.0 |
| 788 | 2,3-dimethyl-2-pentanol | 4911-70-0 | 59 | 100.0 | 100.0 |
| 857 | 2,5-dimethyl-2-hexanol | 3730-60-7 | 59 | 100.0 | 100.0 |
| 1029 | 2-ethyl-1-hexanol | 104-76-7 | 57 | 87.5 | 80.0 |
| Ketones | | | | | |
| 488 | acetone | 67-64-1 | 58 | 100.0 | 100.0 |
| 596 | 2-butanone | 78-93-3 | 72 | 100.0 | 100.0 |
| 685 | 2-pentanone | 107-87-9 | 86 | 100.0 | 100.0 |
| 787 | 3-hexanone | 589-38-8 | 100 | 100.0 | 100.0 |
| 788 | 2-hexanone | 591-78-6 | 58 | 100.0 | 100.0 |
| 889 | 2-heptanone | 110-43-0 | 58 | 100.0 | 100.0 |
| 991 | 2-octanone | 111-13-7 | 58 | 100.0 | 100.0 |
| 657 | methyl butanone | 563-80-4 | 86 | 100.0 | 100.0 |
| 711 | 3,3-dimethyl-2-butanone | 75-97-8 | 100 | 91.7 | 87.5 |
| 738 | 4-methyl-2-pentanone | 108-10-1 | 58 | 100.0 | 100.0 |
| 750 | 2-methyl-3-pentanone | 565-69-5 | 57 | 100.0 | 97.5 |
| 752 | 3-methyl-2-pentanone | 565-61-7 | 57 | 100.0 | 100.0 |
| 781 | 4,4-dimethyl-2-pentanone | 590-50-1 | 58 | 100.0 | 100.0 |
| 791 | 2,4 dimethyl-3-pentanone | 565-80-0 | 71 | 100.0 | 100.0 |
| 1075 | acetophenone | 98-86-2 | 105 | 79.2 | 77.5 |
| 1183 | m-methylacetophenone | 585-74-0 | 119 | 79.2 | 75.0 |
| 1197 | p-methylacetophenone | 122-00-9 | 119 | 62.5 | 75.0 |
| 896 | cyclohexanone | 108-94-1 | 55 | 100.0 | 100.0 |
| 587 | 2,3-butanedione | 431-03-8 | 86 | 100.0 | 100.0 |
| 688 | 2,3-pentanedione | 600-14-6 | 100 | 91.7 | 95.0 |
| 826 | 3-methyl-2,4-pentanedione | 815-57-6 | 72 | 100.0 | 100.0 |
| 671 | 3-methyl-3-buten-2-one | 814-78-8 | 84 | 100.0 | 100.0 |
| 824 | 5-methyl-3-hexen-2-one | 5166-53-0 | 97 | 100.0 | 97.5 |
| 839 | 3-methyl-3-penten-2-one | 565-62-8 | 98 | 87.5 | 92.5 |
| 960 | 3,4-dimethyl-3-hexen-2-one | 1635-02-5 | 55 | 79.2 | 80.0 |
| 710 | 3-hydroxy-2-butanone | 513-86-0 | 88 | 83.3 | 77.5 |

(continued)

| Sulfur-containing volatiles | | | | | |
| --- | --- | --- | --- | --- | --- |
| 417 | methanethiol | 74-93-1 | 47 | 37.5 | 52.5 |
| 518 | dimethylsulfide | 75-18-3 | 62 | 62.5 | 65.0 |
| 747 | dimethyldisulfide | 624-92-0 | 94 | 66.7 | 67.5 |
| 913 | dimethylsulfone | 67-71-0 | 79 | 95.8 | 92.5 |
| 786 | 3-methylthiophene | 616-44-4 | 97 | 100.0 | 100.0 |
| Other volatile compounds | | | | | |
| 1028 | m-cymene | 535-77-3 | 119 | 95.8 | 100.0 |
| 1034 | p-cymene | 99-87-6 | 119 | 100.0 | 100.0 |
| 1097 | dehydro-p-cymene | 1195-32-0 | 117 | 83.3 | 85.0 |
| 524 | methyl acetate | 79-20-9 | 74 | 45.8 | 65.0 |
| 613 | ethyl acetate | 141-78-6 | 70 | 100 | 100 |
| 620 | tert-butyl ethyl ether | 637-92-3 | 59 | 62,5 | 50 |

### • Identification of volatile organic compounds issued from external contamination

Some compounds were suspected to arise artefactually from analysis conditions and/or from external contamination. The analysis of the atmosphere of the room where collection took place, the stabilizing solution employed for samples, and the septa and fiber PDMS/CAR were the sources of 29 compounds (Table 4). The extraction buffer was analyzed without saliva under the same analytical conditions. PDMS-CAR desorption was analyzed using mineral water. The atmosphere of the sample collection room location was analyzed by SPME-GC-MS.

**Table 4. Volatile compounds possibly arising from external and/or analysis conditions.**

| Possible contamination origins | Compounds |
| --- | --- |
| Septa / PDMS-CAR fiber | 2-methylpentane |
| | 3-methylpentane |
| Extraction buffer | acrylonitrile |
| | ethyl isocyanate |
| | propane nitrile |
| | 3-butenenitrile |
| | butanenitrile |
| | pentanenitrile |
| | hexanenitrile |
| | benzene isocyanato |
| | heptanenitrile |
| | benzonitrile |
| Room atmosphere | methyl acetate |
| | ethyl acetate |
| PDMS-CAR fiber/tobacco | 2-methylfuran |
| | 3-methylfuran |
| | 2-ethylfuran |

(continued)

| Possible contamination origins | Compounds |
|---|---|
| Room atmosphere/extraction buffer | 2-methylpropene |
| | tert-butyl ethyl ether |
| | benzene |
| | toluene |
| | ethylbenzene |
| | *m*-xylene |
| | *m*-cymene |
| | *p*-cymene |
| | benzene, (2-methylpropenyl) |
| | dehydro p-cymene |
| | *m*-methyl-acetophenone |
| | *p*-methyl-acetophenone |

Aromatic compounds and esters were predominant in the room-collection atmosphere, whilst nitriles and other nitrogen-containing compounds were sourced from the buffer solution, 2- and 3-methylpentanes, and some furan compounds were known to be derived from fiber, septa and the column. However, some of these molecules could also arise from the smoking of tobacco products. The decision to not retain these molecules in the statistical analyses was made since it was difficult to estimate the distribution of the differing origins for each of them. Therefore, all analyses were performed on the remaining 78 VOCs (see Table 1).

The principle of SPME involves equilibration of analytes between the sample matrix and an organic polymeric phase coated on a fused-silica fiber. During saliva sample collection, it was impossible to obtain the same salivary volume from each participant. A complementary test to study variability of the saliva sample collection from 4 healthy participants showed fluctuations, with an increase in the global abundance of volatile compounds at a diurnal time-point of almost 5 pm in the afternoon (data not shown). Therefore, spectral data acquired could show large variations of the global abundance of VOCs linked with salivary contents and with the time of the saliva collection. These observations led us to determine the minimum amount of saliva collected under which the analysis of VOCs was not possible, and since any volatile compound identified could be used as an internal standard, the calculation of all ratios served as the best possible option to compare VOCs under the same conditions.

Amongst the 1,767 ratios calculated from the 78 VOCs, 6 were selected by factorial discriminant analysis (FDA) of the 54 training samples in order to distinguish between the control and cancer test groups. In order to compare mean values of the two groups of samples, the non-parametric Mann-Whitney test was employed (Table 5).

**Table 5. Mann-Whitney test p and Z values for the 6 selected discriminatory molecular concentration ratios.**

| Ratios | U | p value | Z value |
|---|---|---|---|
| 2-methyl-2-butenal /methyl butanone | 180.5 | 0.00003 | 4.153 |
| 3-methyl-3-buten-2-one/2-methyl-2-butenal | 627 | 0,042 | 2.039 |
| 3-methyl-2-pentanone/butanal | 231 | 0,0006 | 3.453 |
| 2,4 dimethyl-3-pentanone, /methyl butanone | 357.5 | 0,09 | 1.699 |
| 5-methyl-3-hexen-2-one/acetone | 703 | 0,002 | 3.092 |
| dimethyl sulfide/1-propanol | 575 | 0,18 | 1.317 |

Four ratios had significantly different mean values (p< 0.05) between the two groups. For the 2,4-dimethyl-3-pentanone/methyl butanone and dimethyl sulfide/propan-1-ol ratios, such values were not significantly different. For the latter ratio, this may be explicable by the observation that dimethyl sulfide was the only compound which was not detected in all samples. The (2,4-dimethyl-3-pentanone /methyl butanone) ratio was the least discriminant of the 6 determinate ratios, and this may arise from higher intra-individual variations of the 2,4 dimethyl-3-pentanone concentrations within each group.

The results of FDA gave a linear coefficient for each ratio (R1 to R6). These coefficients allowed the construction of the factorial axis via a linear combination (LC) (Table 6).

**Table 6. The 6 ratios selected by FDA with their coefficient values within the linear combination (LC) of the discrimination axis.**

| Ratio | Code | Coefficient value of the linear combination |
|---|---|---|
| 2-methyl-2-butenal/ methyl butanone | R1 | -4.88 |
| 3-methyl-3-buten-2-one/2-butenal, 2-methyl- | R2 | 1.21 |
| 3-methyl-2-pentanone/butanal | R3 | -1.51 |
| 2,4-dimethyl-3-pentanone/ methylbutanone | R4 | -4.33 |
| 5-methyl-3-hexen-2-one/acetone | R5 | 70.49 |
| dimethyl sulfide/1-propanol | R6 | 21.01 |
| | | 2.51 (constant) |

This LC is provided in equation (1).

$$LC = 2.51 - 4.88R1 + 1.21R2 - 1.51R3 - 4.33R4 + 70.49R5 + 21.01R6 \quad (1)$$

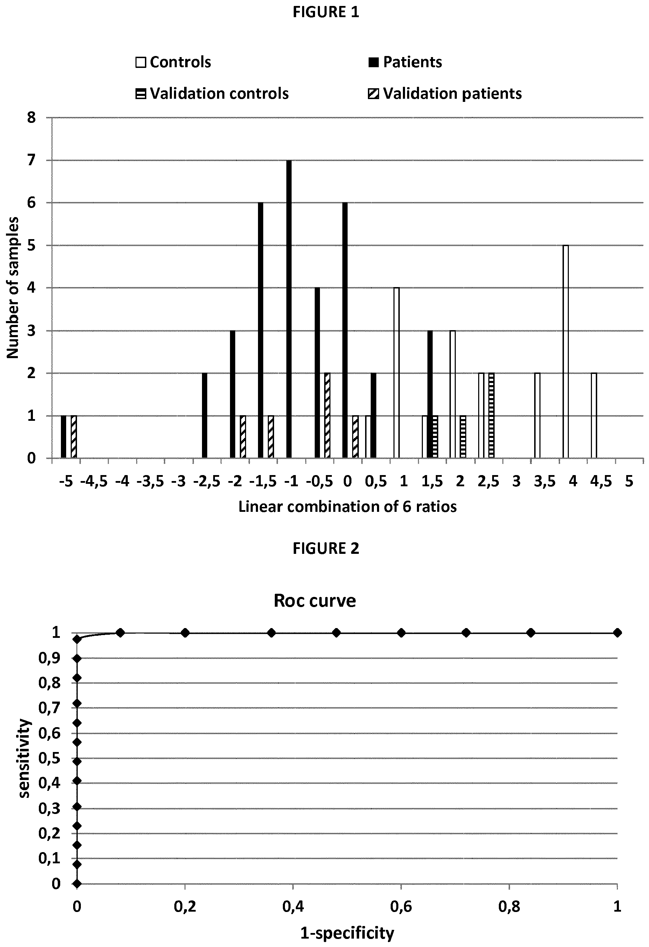

The classification model with the 6 ratios correctly classified 93.75% of the samples according to the factor "tumor"; all 10 validation samples (4 controls and 6 tests) were tested in this discriminant model (Figure 1).

The applicability of determining the LC value in saliva for discriminating between test and control participants was assessed with the ROC curve strategy (Figure 2). Using a value of 0.60 as a cut-off value for LC, the sensitivity of this model was 92.5%, and the specificity was 95.8%. All the results are illustrated in table 7:

**Table 7: Sensitivity and specificity related to cut-off values**

| LC (cut-off) | TP | FN | TN | FP | Sensitivity | 1-Specificity |
|---|---|---|---|---|---|---|
| -0,5 | 29 | 11 | 24 | 0 | 0,725 | 0,000 |
| 0 | 35 | 5 | 24 | 0 | 0,875 | 0,000 |
| 0,2 | 36 | 4 | 24 | 0 | 0,9 | 0,000 |
| 0,4 | 37 | 3 | 24 | 0 | 0,925 | 0,000 |
| 0,5 | 37 | 3 | 23 | 1 | 0,925 | 0,042 |
| 0,6 | 37 | 3 | 23 | 1 | 0,925 | 0,042 |
| 0,7 | 37 | 3 | 22 | 2 | 0,925 | 0,083 |
| 0,9 | 37 | 3 | 21 | 3 | 0,925 | 0,125 |
| 1,1 | 39 | 1 | 19 | 5 | 0,975 | 0,208 |
| 1,2 | 40 | 0 | 19 | 5 | 1 | 0,208 |

In the case of using this linear combination alone for diagnosis, the cut-off of 1.2 is the most relevant since there are no false negatives; the associated sensitivity is 100% and the specificity 79.17%.

**8. Comparative studies**

The two statistical models disclosed in the European patents EP 2 321 430 B1 were compared to the method of diagnosing oral cancer or assessing risk of developing oral cancer in a subject according to the invention.

In EP 2 321 430 B1, among the 108 volatile molecules identified in saliva sample of the 45 subjects, 49 compounds were found significant to separate the group "tumor" from the reference group. In statistical model 1, among the ratios calculated from these VOCs, 5 were selected by factorial discriminant analysis (FDA) in order to distinguish between the control and cancer test groups: R1=3-methyl-2-pentanone/methyl butanone, R56=butanal/hexanal, R260=hexanenitrile/1-propanol, R266=2-propanol/(cis) 1,2-dimethyl cyclopropane and R269=Phenol/2,3-butanedione. Only the three ratios R1, R260 and R266 were found indicative of oral cancer suffering patients. A linear coefficient for each ratio was obtained using FDA analysis. These coefficients allowed the construction of a factorial axis via a linear combination (LC) provided in the following equation: LC = 1.8277-7.3472*R1 - 0.125*R266 - 14.2293*R260 + 1.2050*R269 + 8.883*R56.

The saliva samples of both the 40 subjects who were diagnosed with a squamous cell carcinoma of head and neck (HNSCC) and the 24 healthy participants were assayed with the linear combination of statistical model 1 of EP 2 321 430 B1. Using a value of 0.6 as a cut-off value for LC, the sensitivity of this model is 15% and the specificity is 58.3% (true positive samples (TP)=6; false negative samples (FN)=34; true negative samples (TN)=14 and false positive samples (FP)=10).

This linear combination does not allow samples to be classified according to their group, i.e. HNSCC patient or healthy participant. Moreover, hexanenitrile was identified as a VOC varying with experimental conditions.

In the statistical model 2, 98 volatile molecules identified in saliva sample of the 45 subjects, 49 compounds were found significant to separate the group "tumor" from the reference group. Five ratios were selected by factorial discriminant analysis (FDA) in order to distinguish between the control and cancer test groups: R1=butanal/2-butanone, R2=3-buten-2-one, 3-methyl/acetone, R3=1-propene, 2-methyl/pentanal, R4=3-hexen-2-one, 5-methyl/2-butenal, 2-methyl and R5=acrylonitrile/1-propanol. A linear coefficient for each ratio was obtained using FDA analysis. These coefficients allowed the construction of a factorial axis via a linear combination (LC) provided in the following equation: LC = -3.92 + 6.25*R1 + 24.22*R2 + 0.168*R3 + 1.62*R4 + 11.15*R5. The saliva samples of both the 40 subjects who were diagnosed with a squamous cell carcinoma of head and neck (HNSCC) and the 24 healthy participants were assayed with the linear combination of statistical model 2 of EP 2 321 430 B1. Results obtained for the 64 samples are the following ones (table 8):

**Table 8: Sensitivity and specificity related to cut-off values**

| LC (cut-off) | TP | FN | TN | FP | Sensitivity | 1-Specificity |
|---|---|---|---|---|---|---|
| 0,5 | 29 | 11 | 20 | 4 | 0,725 | 0,167 |
| 0,6 | 30 | 10 | 18 | 6 | 0,75 | 0,250 |
| 0,7 | 31 | 9 | 18 | 6 | 0,775 | 0,250 |
| 0,9 | 32 | 8 | 18 | 6 | 0,8 | 0,250 |
| 1,1 | 32 | 8 | 17 | 7 | 0,8 | 0,292 |
| 1,3 | 34 | 6 | 14 | 10 | 0,85 | 0,417 |
| 1,5 | 35 | 5 | 12 | 12 | 0,875 | 0,500 |
| 2 | 36 | 4 | 12 | 12 | 0,9 | 0,500 |

Using a value of 0.9 as a cut-off value for LC, the sensitivity of this model is 80% and the specificity is 75% (true positive sample (TP)=32; false negative sample (FN)=8; true negative samples (TN)=18 and false positive samples(FP)=6).

It is impossible to obtain a classification excluding false positives with acceptable specificity, thus discarding this linear combination. Moreover, 1-propene, 2-methyl and acrylonitrile were identified as VOCs varying with experimental conditions.

The comparative studies can be summarized as follows:

**Table 9: comparative studies on 64 samples for a cut-off value of 0.6**

|  | Statistical model 1 of EP 2 321430 B1 | Statistical model 2 of EP 2 321 430 B1 | Statistical model of the present invention |
|---|---|---|---|
| Sensitivity | 15% | 75% | 92.5% |
| Specificity | 58% | 75% | 95.8% |

As a conclusion, the statistical model of the present invention is robust and allows diagnostic of subject suffering from HNSCC.

**Claims**

1. An *in vitro* method of diagnosing oral cancer or assessing risk of developing oral cancer in a subject, comprising :

   a) determining the concentration of at least the seven following volatile organic compounds (VOCs) in a saliva sample obtained from the subject:

   - 2-methyl-2-butenal (CAS number 497-03-0),
   - methyl butanone (CAS number: 563-80-4),
   - 3-methyl-3-buten-2-one (CAS number 814-78-8),
   - 3-methyl-2-pentanone (CAS number 565-61-7),
   - butanal (CAS number 123-72-8),
   - 5-methyl-3-hexen-2-one (CAS number 5166-53-0), and
   - acetone (CAS number 67-64-1),and

   b) calculating the four following ratios :

   - R1 = 2-methyl-2-butenal (CAS number 497-03-0) /methyl butanone (CAS number: 563-80-4),
   - R2 = 3-methyl-3-buten-2-one (CAS number 814-78-8) /2-methyl-2-butenal (CAS number 497-03-0),
   - R3 = 3-methyl-2-pentanone (CAS number 565-61-7) / butanal (CAS number 123-72-8), and
   - R5 = 5-methyl-3-hexen-2-one (CAS number 5166-53-0) / acetone (CAS number 67-64-1),

   wherein said ratios are compared to reference ratios.

2. The method according to claim 1, wherein step a) further comprises determining the concentration of the three following VOCs:

   - 2,4 dimethyl- 3-pentanone (CAS number 565-80-0),
   - dimethyl sulfide (CAS number 75-18-3), and
   - 1-propanol (CAS number 71-23-8), and
   wherein the step b) further comprises calculating the two following ratios:

   - R4 = 2,4 dimethyl- 3-pentanone (CAS number 565-80-0) / methyl butanone (CAS number: 563-80-4),and
   - R6 = dimethyl sulfide (CAS number 75-18-3) / 1-propanol (CAS number 71-23-8).

3. The method according to any of claim 1 or 2, wherein the VOCs are detected in the volatile fraction of the saliva sample.

4. The method according to any of claims 1 to 3, wherein the saliva sample is stabilized using a preservation solution comprising guanidinium thiocyanate, and/or sodium azide and/or ammonium sulfate, and/or monosodium citrate, and/or trisodium citrate dehydrate.

5. The method according to any one of claims 1 to 4, wherein the VOCs are detected using mass spectrometry.

6. The method according to claim 5, wherein the mass spectrometry is SPME-GC-MS.

7. The method of claim 2, further comprising the step of calculating a linear combination (LC) according to the following formula:

$$LC = 2.51 - A \times (R1) + B \times (R2) - C \times (R3) - D \times (R4) + E \times (R5) + F \times (R6),$$

wherein A, B, C, D, E and F are constants.

8. The method according to claim 7, wherein A = 4.88, B = 1.21, C = 1.51, D = 4.33, E = 70.49 and F = 21.01.

9. The method according to claim 6 or 7, wherein oral cancer or increased risk of developing oral cancer is diagnosed if $LC \leq +1.5$.

10. The method according to any one of claims 1 to 9, wherein the diagnosis or assessing the risk of oral cancer has a specificity of at least 95% and a sensitivity of at least 92 %.

11. Use of a kit for the implementation of the method according to any one of claims 1 to 10, wherein said kit comprises a sterile device to collect a saliva sample, a collection reagent, at least one preservation solution, and optionally, an electronic sensor.

## FIGURE 1

## FIGURE 2

FIGURE 3

TIC:SALI31B.D

Europäisches
Patentamt

European
Patent Office

Office européen
des brevets

# EUROPEAN SEARCH REPORT

Application Number

EP 19 00 0185

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHIGEYAMA HIROYO ET AL: "Identification of volatile metabolites in human saliva from patients with oral squamous cell carcinoma via zeolite-based thin-film microextraction coupled with GC-MS", JOURNAL OF CHROMATOGRAPHY B: BIOMEDICAL SCIENCES & APPLICATIONS, ELSEVIER, AMSTERDAM, NL, vol. 1104, 6 November 2018 (2018-11-06), pages 49-58, XP085570552, ISSN: 1570-0232, DOI: 10.1016/J.JCHROMB.2018.11.002 * page 50 - page 57; figures; tables 2-4 * | 1-11 | INV. G01N33/574 G01N33/68 |
| A | M BOUZA ET AL: "Exhaled breath and oral cavity VOCs as potential biomarkers in oral cancer patients", JOURNAL OF BREATH RESEARCH, vol. 11, no. 1, 1 March 2017 (2017-03-01), page 016015, XP055635776, DOI: 10.1088/1752-7163/aa5e76 * page 2 - page 8; table 2 * | 1-11 | |
| X,D | WO 2010/015607 A2 (INST CLINIDENT [FR]; CHAUBRON FRANCK [FR]) 11 February 2010 (2010-02-11) * see Example 10: Diagnostic test based on the ratios of specific organic molecules; page 27 - page 36; claims 1-27; figures; examples; tables 3-5 * | 1-11 | TECHNICAL FIELDS SEARCHED (IPC) G01N |
| X | WO 2011/015589 A1 (INST CLINIDENT [FR]; CHAUBRON FRANCK [FR]) 10 February 2011 (2011-02-10) * claims; example 5; tables 4, 5 * | 1-11 | |

-/--

The present search report has been drawn up for all claims

| Place of search | Date of completion of the search | Examiner |
|---|---|---|
| Munich | 29 October 2019 | Gonçalves Mauger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 19 00 0185

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AIHUA ZHANG ET AL: "Saliva Metabolomics Opens Door to Biomarker Discovery, Disease Diagnosis, and Treatment", APPLIED BIOCHEMISTRY AND BIOTECHNOLOGY ; PART A: ENZYME ENGINEERING AND BIOTECHNOLOGY, HUMANA PRESS INC, NEW YORK, vol. 168, no. 6, 13 September 2012 (2012-09-13), pages 1718-1727, XP035141040, ISSN: 1559-0291, DOI: 10.1007/S12010-012-9891-5 * page 723 - page 725 * ----- | 1-11 | |
| X | METZGER K ET AL: "Lipoxygenase products in human saliva: Patients with oral cancer compared to controls", FREE RADICAL BIOLOGY AND MEDICINE, ELSEVIER INC, US, vol. 18, no. 2, 1 January 1995 (1995-01-01), pages 185-194, XP002596552, ISSN: 0891-5849 * page 186 - page 194 * ----- | 1-11 | |
| A | MACIEJ MILANOWSKI ET AL: "Saliva - Volatile Biomarkers and Profiles", CRITICAL REVIEWS IN ANALYTICAL CHEMISTRY., vol. 47, no. 3, 1 December 2016 (2016-12-01), pages 251-266, XP055636680, US ISSN: 1040-8347, DOI: 10.1080/10408347.2016.1266925 * page 255 - page 263; table 5 * ----- | 1-11 | TECHNICAL FIELDS SEARCHED (IPC) |

The present search report has been drawn up for all claims

| Place of search | Date of completion of the search | Examiner |
|---|---|---|
| Munich | 29 October 2019 | Gonçalves Mauger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 00 0185

This annex lists the patent family members relating to the patent documents cited in the above-mentioned European search report.
The members are as contained in the European Patent Office EDP file on
The European Patent Office is in no way liable for these particulars which are merely given for the purpose of information.

29-10-2019

| Patent document cited in search report | Publication date | Patent family member(s) | | Publication date |
|---|---|---|---|---|
| WO 2010015607 A2 | 11-02-2010 | CA | 2733246 A1 | 11-02-2010 |
| | | DK | 2321430 T3 | 20-01-2014 |
| | | EP | 2321430 A2 | 18-05-2011 |
| | | ES | 2441586 T3 | 05-02-2014 |
| | | US | 2011143962 A1 | 16-06-2011 |
| | | US | 2014065600 A1 | 06-03-2014 |
| | | WO | 2010015607 A2 | 11-02-2010 |
| WO 2011015589 A1 | 10-02-2011 | EP | 2462448 A1 | 13-06-2012 |
| | | US | 2012126111 A1 | 24-05-2012 |
| | | WO | 2011015589 A1 | 10-02-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2321430 B1

**Non-patent literature cited in the description**

- *Clin. Cancer Res.,* 2008, vol. 14, 6246-6252